# EUROPEAN PATENT SPECIFICATION

(11) **EP 3 504 988 B1**
(45) Date of publication and mention of the grant of the patent: **07.04.2021**
(21) Application number: 16914236.1
(22) Date of filing: 26.08.2016
(51) Int. Cl.: A24F 47/00, A24F 40/30, A24F 40/485

(54) **NON-COMBUSTION FLAVOR INHALER**
VERBRENNUNGSFREIER AROMAINHALATOR
INHALATEUR D'ARÔME SANS COMBUSTION

(43) Date of publication of application: 03.07.2019
(73) Proprietor: Japan Tobacco, Inc., Tokyo 105-8422 (JP)
(72) Inventor: ITO, Kenji, Tokyo 130-8603 (JP)
(74) Representative: Hoffmann Eitle
(86) International application number: PCT/JP2016/075035
(87) International publication number: WO 2018/037562

(56) References cited:
- WO-A1-2010/095660
- WO-A1-2014/156537
- WO-A1-2016/121143
- DE-A1-102014 114 308
- US-A1- 2016 073 693

## Description

### Technical Field

The present invention relates generally to a non-combustion flavor inhaler.

### Background Art

Currently, a non-combustion flavor inhaler (hereinafter also referred to simply as a flavor inhaler) which generates a flavor without combustion and allows a user to inhale its flavor has been put to practical use.

A flavor inhaler comprising an aroma substance tank disposed on its outer circumferential surface to allow the user to enjoy the fragrance more clearly is known (JP 2013-521074 A).

DE 102014 114308 A1 is related to the preamble of claim 1.

### Summary of Invention

### Technical Problem

The present invention provide a non-combustion flavor inhaler capable of stimulating a sense of smell by wafting a flavor to the user's nose only at non-puffing and stimulating senses of taste and smell by allowing a flavor to be inhaled in a user's oral cavity at puffing.

In this case, "at puffing" means the time when the user holds the mouthpiece end side of the flavor inhaler in the mouth and inhales. In addition, "at non-puffing" means the time when the user does not hold the mouthpiece end side of the flavor inhaler in user's mouth or inhale.

### Means for Solving the Problem

According to the present invention, there is provided a non-combustion flavor inhaler having the features of claim 1.

### Brief Description of Drawings

FIG. 1 is a schematic cross-sectional view illustrating a non-combustion flavor inhaler according to a first embodiment.
FIG. 2 is an enlarged cross-sectional view illustrating an atomizer unit shown in FIG. 1.
FIG. 3 is an enlarged cross-sectional view illustrating a flavor generating unit of an atomizer unit in the flavor inhaler according to the first embodiment.
FIG. 4 is an enlarged cross-sectional view illustrating a flavor generating unit of an atomizer unit in a flavor inhaler according to a second embodiment.
FIG. 5 is an enlarged cross-sectional view illustrating a flavor generating unit of an atomizer unit in a flavor inhaler according to a third embodiment.
FIG. 6 is an enlarged cross-sectional view illustrating a flavor generating unit of an atomizer unit in a flavor inhaler according to a fourth embodiment.
FIG. 7 is an enlarged cross-sectional view illustrating a flavor generating unit of an atomizer unit in a flavor inhaler according to a fifth embodiment.
FIG. 8 is an enlarged cross-sectional view illustrating a flavor generating unit of an atomizer unit in a flavor inhaler according to a sixth embodiment.

### Mode for Carrying Out the Invention

A non-combustion flavor inhaler according to the embodiments will be described below in detail.

### (First Embodiment)

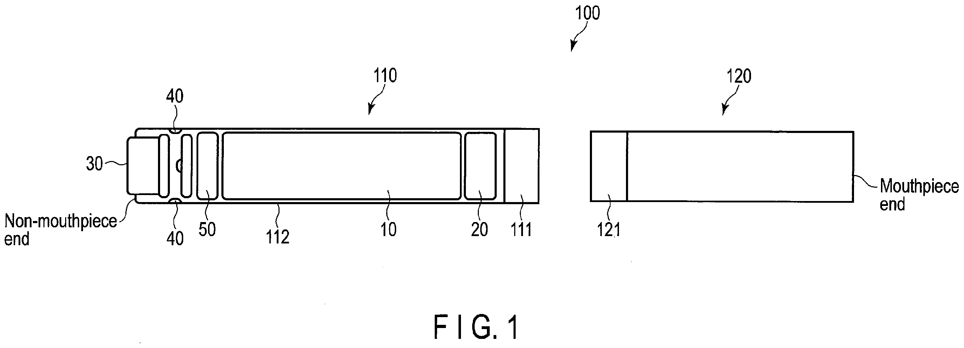

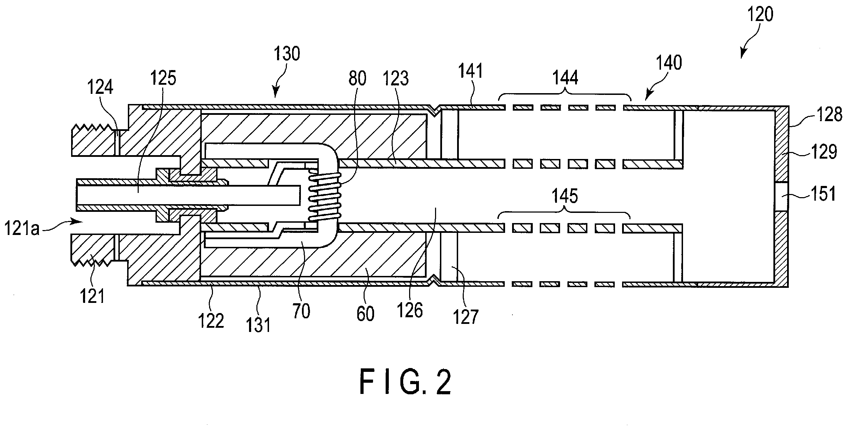

A first embodiment will be described with reference to FIG. 1 and FIG. 2. FIG. 1 is a schematic cross-sectional view illustrating a non-combustion flavor inhaler 100 according to the first embodiment. FIG. 2 is an enlarged cross-sectional view illustrating an atomizer unit 120 shown in FIG. 1.

In FIG. 1, the non-combustion flavor inhaler 100 comprises a power supply unit 110 and an atomizer unit 120 disposed on, for example, the mouthpiece end side of the power supply unit 110. The power supply unit 110 has a non-mouthpiece end and includes a first connection portion 111 at the other end. The atomizer unit 120 has a mouthpiece end 128 and includes a second connection section 121 at the other end. For example, a female thread and a male thread which can be screwed with each other are formed at the first connection portion 111 and the second connection portion 121, respectively. The power supply unit 110 and the atomizer unit 120 are detachable from each other via the first connection portion 111 and the second connection unit 121.

As shown in FIG. 2, the atomizer unit 120 comprises, for example, a cylindrical second housing 122. A mouthpiece end 128 defined by sealing an opening end with, for example, a wall portion 129 integrated with the second housing 122, is formed on one of ends of the cylindrical second casing 122, and the second connection portion 121 is formed on the other end. In the second housing 122, the aerosol generation unit 130 and a flavor generation unit 140 are arranged in this order from the first connection portion side.

The female thread, which is the second connection portion 121, includes a recess 121a hollowed out in the axial direction. A plurality of air intake holes 124 for introducing outside air into a first flow path are opened in the second connection portion 121 so as to reach the recess 121a from its outer circumferential surface. A fourth flow path 125 formed of a pipe body, which penetrates the central portion of the second connection portion 121 and communicates with the inside of the power supply unit 110, is provided in the second housing 122. A cylindrical first partition wall 123 extending from the second connection portion 121 toward the mouthpiece end and communicating with the fourth flow path 125 is provided in the center of the second housing 122. A first flow path 126 is formed in the first partition wall 123. A first inhalation hole 151 to allow a user to inhale the gas in the flavor inhaler 100 is opened at the center of the wall portion 129 of the mouthpiece end 128 of the second housing 122. The first inhalation hole 151 communicates with the first flow path 126.

The aerosol generation unit 130 is disposed on, for example, the second connection portion 121 side in the second housing 122. An aerosol source storage section 131, a holding body 60, an absorber 70, and an aerosol generation mechanism 80 are disposed in the second housing 122. For example, the aerosol source storage section 131 is formed in a cylindrical shape in which an outer wall is defined by the second housing 122, an inner wall is defined by the first partition wall 123, an end of the non-mouthpiece end side is defined by the second connection portion 121, an end of the mouthpiece end side is defined by a second partition wall 127, to accommodate a cylindrical holding body 60. The holding body 60 is formed of, for example, a porous resin or a foamed resin, and holds and accommodates an aerosol source.

The aerosol source is not particularly limited as long as an aerosol is generated by the aerosol generation mechanism 80 but, for example, an aerosolforming substance such as glycerin or propylene glycol, water, a solvent, ethanol, a plant extract, a natural or artificial flavoring agent (for example, menthol) can be used. The aerosol source may contain a tobacco flavor containing compound or a nicotine containing material.

The absorber 70 is formed, for example, in a U shape, in which both ends are held in the aerosol source storage section 131 and a central portion is disposed close to the tip end of a tube body which is the fourth flow path 125. The absorber 70 is formed of, for example, a bundle of glass fibers, and the liquid aerosol source held by the holding body 60 is moved by a capillary phenomenon.

The aerosol generation mechanism 80 may be any mechanism as long as it generates aerosol from an aerosol source and, for example, a mechanism generating the aerosol by heating the aerosol source can be used. The aerosol generation mechanism 80 is, for example, a first heating element. The first heating element 80 is composed of, for example, a coil heater wound around the absorber 70, which is formed of, for example, stainless steel, copper, a copper alloy, a nickelchromium alloy, or a superalloy. The first heating element 80 is heated to a temperature at which an aerosol can be generated from an aerosol source, for example, 150°C to 350°C. The first heating element 80 can be used, for example, of a structure in which the voltage applied to the first heating element 80 is adjusted by a dial operation attached to the flavor inhaler.

The flavor generation unit 140 is disposed, for example, between the aerosol generation unit 130 in the second housing 122 and the mouthpiece end and comprises a flavor source storage section 141. In the flavor source storage section 141, for example, an outer wall is the second housing 122, the inner wall is the first partition wall 123, and the end of the second connection portion side is surrounded by the second partition wall 127, and the mouthpiece end side is surrounded by a partition wall located on the mouthpiece end side, and a flavor source which generates flavor is stored. A second flow path 144 composed of at least one hole communicating with the outside, for example, a plurality of holes, is provided in the second housing 122 portion. A third flow path 145 composed of a plurality of holes communicating with the first flow path 126 is provided in the cylindrical first partition wall 123 portion. The second flow path 144 and the third flow path 145 preferably comprise flavor-permeable members (not shown). The flavor-permeable member is, for example, a porous membrane having a large number of fine pores opened therein, for example, to prevent a granular flavor source from flowing out to the outside.

The flavor source in the flavor source container 141 may be a flavor source which can adjust the amount or type of flavor generated by physical disruption. For example, the flavor source may be a plurality of capsules containing an artificial or natural flavoring agent. In this case, the capsules are stored in the flavor source storage section 141, and the portion of the second housing 122 where the second flow path 144 is provided is formed of a flexible material. In such a configuration, the user can crush the capsules which are the flavor source in the flavor source storage section 141 and generate a flavor by pressing the portion of the second housing 122 where the second flow path 144 is provided with a finger or the like. The user can adjust the flavor components and amounts of the generated flavor by selecting the type and amount of capsules to be crushed. The user may generate the flavor included in the capsules by crushing with stimulation such as ultrasonic waves instead of pressing the capsules with a finger or the like. For example, the flavor source may be formed in a shape of highly disintegratable granules, tablets or the like, and the amount of flavor generated may be adjusted by grinding the flavor source having excipients or coating materials added as needed.

The other flavor sources contain solid substances which allow air to permeate through the inside of the flavor source storage section, for example, plant origin, plant extract, nicotine, menthol, and natural or artificial flavoring agents. More specifically, examples include shredded tobacco, shaped bodies of tobacco materials in granules, shaped bodies of various flavoring agents in granules, shaped bodies of tobacco materials in sheet form, and plant origins other than tobacco (for example, mint, herbs, and the like). Besides the shaped bodies, the examples include, for example, granular adsorbents impregnated with plant extracts, and natural or artificial flavoring agents.

As shown in FIG. 1, for example, the power supply unit 110 described above comprises a first housing 112 in a cylindrical shape. A switch 30, a light emitting element 40, a control circuit 50, a power supply 10, and a sensor 20 are arranged in this order from the non-mouthpiece end side, in the first casing 112.

The power supply 10 is, for example, a lithium ion secondary battery, and is electrically connected to the sensor 20, the light emitting element 40, the control circuit 50, and the first heating element 80. The sensor 20 can use a piezoelectric element which detects the airflow in the flavor inhaler 100 generated by the user's inhaling operation, for example, a negative pressure generated by inhaling gas toward the mouthpiece end. The switch 30 is arranged on, for example, the non-mouthpiece end and sets the power supply 10 of the flavor inhaler 100 to be turned on or off by a push button operated by pushing in the longitudinal direction of the power supply unit 110. The light emitting element 40 is, for example, a light emitting diode embedded on the non-mouthpiece end side of the outer circumferential surface of the first housing 112 and notifies the user of the state of the flavor inhaler 100 by a light emission pattern or a light emission color. More specifically, the light emitting element 40 is turned on when the power supply 10 is in the ON state, and is turned off when the power supply 10 is in the OFF state. The control circuit 50 is connected to, for example, the power supply 10, the sensor 20, the switch 30, the light emitting element 40, and the first heating element 80, and performs feedback control of the first heating element 80 based on, for example, detection of the sensor 20 or controls the light emission pattern of the light emitting element 40.

Next, the operation of the flavor inhaler 100 having the above-mentioned configuration will be described with reference to FIG. 1 to FIG. 3. The user turns on the power supply 10 of the flavor inhaler 100 with the switch 30 and uses it with the mouthpiece end side in the mouth.

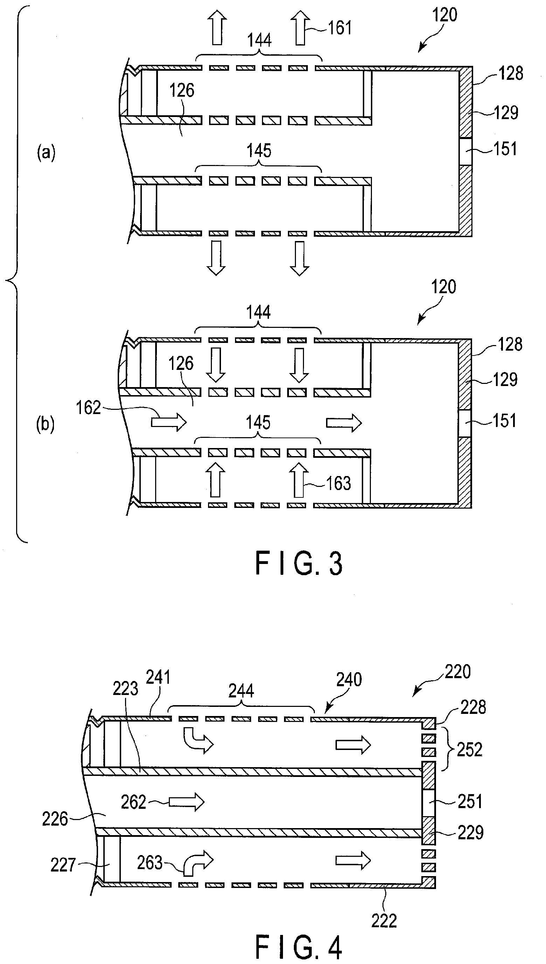

As shown in (a) of FIG. 3, when the user does not inhale the gas inside the flavor inhaler 100 (at non-puffing), flavor 161 flows to the outside from the flavor source stored in the flavor source storage section 141 through the second flow path 144. A user holding the flavor inhaler 100 in the mouth can taste the flavor 161 with a sense of smell since the flowing flavor 161 wafts in the nose.

As shown in (b) of FIG. 3, when the user inhales air in the flavor inhalation device 100 (at puffing), the sensor 20 detects the inhalation operation, and the control signal is output from the control circuit 50 to which the detection signal is input to the first heating element 80, to heat the first heating element 80. The aerosol is generated from the aerosol source held in the absorber 70 by heating of the first heating element 80. In addition, as the user performs the inhaling operation, air flows into the first flow path 126 through the air intake hole 124 and the fourth flow path 125 of the aerosol generation unit. At this time, a first airflow 162 to flow toward the first inhalation hole 151 is generated in the first flow path 126. The aerosol generated from the aerosol source is urged to flow to the first inhalation hole 151 while accompanied by the first airflow 162. At the same time, since the pressure inside the flavor source storage section 141 communicating through the first flow path 126 and the third flow path 145 becomes a negative pressure, a second airflow 163 by which external air flows to the first flow path 126 through the second flow path 144, the flavor source storage section 141, and the third flow path 145 is generated. The flavor generated from the flavor source in the flavor source storage section 141 flows to the first inhalation hole 151 while accompanied by the second airflow 163. As a result, a gas mixture of the aerosol and the flavor passes through the first inhalation hole 151 and is inhaled in the user's oral cavity.

According to the first embodiment, since the flavor flows from the flavor source in the flavor source storage section 141 to the outside through the second flow path 144 and wafts in the nose at non-puffing, a user holding the flavor inhaler 100 in the mouth can taste its flavor with an olfactory sense. In contrast, at the puffing, the flavor does not flow from the flavor source to the outside through the second flow path 144, and the user can inhale the gas mixture of the flavor and the aerosol generated from the aerosol source in the only oral cavity. As a result, the user can enjoy the stimulation of the senses of taste and smell caused by the flavor and the aerosol in the oral cavity without being exposed to the stimulation of the sense of smell due to the flavor from the nose, that is, without making the nose's sense of smell dull by stimulation from the flavor.

In addition, since the flavor inhaler 100 according to the first embodiment comprises the flavor generation unit 140 separately from the aerosol generation unit 130, the flavor source of the components destroyed at the temperature of the first heating element 80 can be stored in the flavor source storage section 141. As a result, the user can inhale various flavors.

In the first embodiment, various modes to be described below can be adopted in addition to the above-described configuration.

The aerosol source can contain only somatosensory contribution components which contribute to taste and stimulation, and the flavor can be contained only in the flavor source. In this structure, since the aerosol does not contain a component contributing to the flavor, the user can taste the flavor without diffusing the flavor to the surroundings.

The second flow path is preferably provided only on the user's nose side. According to this configuration, the flavor generated from the flavor source can be prevented from unnecessarily diffusing to the surroundings and can efficiently stimulate the only user's sense of smell.

The aerosol generation unit and the flavor generation unit accommodated in the second housing can be configured to be detachable. Thus, for example, when the flavor source is reduced, the flavor generation unit has only to be replaced without replacing two units.

The third flow path is not limited to the flow path on the first partition wall 123 side but may be, for example, opened on the side wall opposed to the mouthpiece end of the flavor source storage section, as a plurality of holes.

In the atomizer unit, the flavor generation unit is disposed closer to the mouthpiece end side than to the aerosol generation unit, but its location is not limited to this. For example, in the atomizer unit, the aerosol generation unit may be disposed closer to the mouthpiece end side than to the flavor generation unit.

The aerosol generation unit is configured to comprise the holding body, the absorber, and the first heating element, but the configuration is not limited to this. For example, a blade heater may be used instead of the coil heater, as the first heating element, and heat the aerosol source which is the shredded tobacco into which the first heating element as the blade heater inserted.

A filter may be further provided in the first flow path communicating with the first inhalation hole. As the filter, a tobacco filter, for example, an acetate filter can be used. A foreign substance can be prevented from entering the user's mouth by providing such a filter in the first flow path.

The first heating element is configured to heat when the user inhales the flavor inhaler, but the configuration is not limited to this. For example, when the first heating element is configured to continuously heat after turning on the power supply, the first heating element can stably generate fragrance.

### (Second Embodiment)

A non-combustion flavor inhaler according to a second embodiment will be described with reference to FIG. 4. FIG. 4 is a cross-sectional view showing a flavor generation unit of an atomizer unit of the flavor inhaler. The flavor inhaler according to the second embodiment is the same as the flavor inhaler shown in FIG. 1 and FIG. 2 explained in the first embodiment, except for the flavor generation unit of the atomizer unit.

A flavor generation unit 240 comprises a first partition wall 223 in a cylindrical shape which reaches from a second connection portion to a mouthpiece end 228 inside a second housing 222 in a cylindrical shape. The mouthpiece end 228 is defined by sealing an open end of the second housing 222 with, for example, a wall portion 229 integrated with the second housing 222. The inside of the first partition wall 223 in the cylindrical shape functions as a first flow path 226. In a flavor source storage section 241, for example, an outer wall is surrounded by the second housing 222, the inner wall is surrounded by the first partition wall 223, the end on the second connection portion side is surrounded by a second partition wall 227, and the other end is surrounded by the mouthpiece end 228, and a flavor source which generates flavor is stored.

A second flow path 244 composed of a plurality of holes communicating with the outside is provided in the second housing 222 portion corresponding to the flavor source storage section 241. A first inhalation hole 251 to allow a user to inhale is opened at the center (i.e., a portion opposed to the first flow path 226) of a wall portion 229 defining the mouthpiece end 228 of the second housing 222. A third flow path 252 composed of a plurality of holes communicating with the outside so as to extend from the flavor source storage section 241 to the mouthpiece end 228, is provided at a portion corresponding to the flavor source storage section 241 of the wall portion 229. The third flow path 252 serves as a second inhalation hole.

Next, the operation of the flavor inhaler having the above-mentioned configuration will be described with reference to FIG. 4. The user turns on a power supply 10 of the flavor inhaler with a switch 30 and uses it with holding the mouthpiece end side in the mouth.

At non-puffing, the flavor flows to the outside from the flavor source stored in the flavor source storage section 241 through the second flow path 244. A user holding the flavor inhaler in the mouth can taste the flavor with the sense of smell since the flowing flavor wafts in the nose.

In contrast, at the puffing, as the user performs the inhaling operation, air flows into the first flow path 226 through the air intake hole 124 and the fourth flow path 125 of the aerosol generation unit. At this time, a first airflow 262 to flow toward the first inhalation hole 251 is generated in the first flow path 226. The aerosol generated from the aerosol source is urged to flow to the first inhalation hole 251 while accompanied by the first airflow 262. At the same time, since the pressure inside the flavor source storage section 241 becomes a negative pressure by the user inhaling through the third flow path 252 serving as a second inhalation hole, a second airflow 263 by which external air flows to the flavor source storage section 241 through the second flow path 244 is generated. Thereby, the flavor generated from the flavor source in the flavor source storage section 241 is urged to flow to the third flow path (second inhalation hole) 252 through the second flow path 244 while accompanied by the second airflow 263. As a result, the aerosol and the flavor are inhaled in the user's oral cavity through the first inhalation hole 251 and the third flow path (second inhalation hole) 252.

Therefore, according to the second embodiment, similarly to the first embodiment, the flavor inhaler has the advantage of stimulating the user's sense of smell by the flavor at non-puffing and stimulating the user's senses of taste and smell by the flavor at puffing.

In addition, providing the flavor-permeable members in the second flow path 244 and the third flow path 252 can prevent the flavor source from flowing into the oral cavity when the user inhales with the flavor inhaler.

### (Third Embodiment)

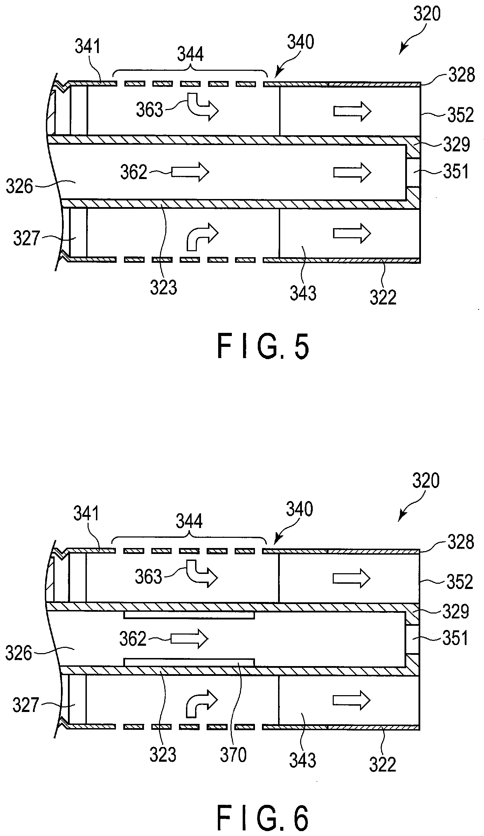

A non-combustion flavor inhaler according to a third embodiment will be described with reference to FIG. 5. FIG. 5 is a cross-sectional view showing a flavor generation unit of an atomizer unit of the flavor inhaler. The flavor inhaler according to the third embodiment is the same as the flavor inhaler shown in FIG. 1 and FIG. 2 explained in the second embodiment, except for the flavor generation unit of the atomizer unit.

A flavor generation unit 340 comprises a first partition wall 323 in a cylindrical shape which reaches from a second connection portion to a mouthpiece end inside a second housing 322 in a cylindrical shape. A mouthpiece end 328 is defined by sealing an open end of the second housing 322 with, for example, a wall portion 329 integrated with the second housing 322. An annular hole 352 is opened in a wall portion 329. The flavor source storage section 341 is, for example, a cylindrical body in which an outer wall is the second housing 322, the inner wall is the first partition wall 323, the end of the second connection portion side is defined by the second partition wall 327, and the other end is defined to be far from the mouthpiece end 328 in a desired distance, and a flavor source is stored. A cylindrical filter 343 having a desired radical thickness is disposed between the second housing 322 and the first partition wall 323 and between the flavor source storage section 341 and the mouthpiece end 328. One end of the filter 343 is located at the flavor source storage section 341 and the other end is located in the annular hole 352 of the wall portion 329 to constitute a third flow path extending from the inside of the flavor source storage section 341 to the mouthpiece end 328. A first flow path 326 is formed inside the first partition wall 323 in the cylindrical shape. At a portion surrounded by an annular hole 352, of the wall portion 329 which defines the mouthpiece end 328 of the second housing 322 (i.e., a portion opposed to the first flow path 326), a first inhalation hole 351 to allow a user to inhale is opened. The other end of the filter 343 located in the annular hole 352 of the wall portion 329 functions as a second inhalation hole.

Preferably, the filter 343 is formed of an airpermeable material and is rough enough to prevent the flavor source in the flavor source storage section 341 from flowing out. The same filter as the tobacco filter, for example, acetate filter can be used as the filter 343.

Next, the operation of the flavor inhaler having the above-mentioned configuration will be described with reference to FIG. 5. The user turns on a power supply 10 of the flavor inhaler with a switch 30 and uses it with holding the mouthpiece end 328 side in the mouth.

At non-puffing, the flavor flows to the outside from the flavor source stored in the flavor source storage section 341 through the second flow path 344. A user holding the flavor inhaler in the mouth can taste the flavor with the sense of smell since the flowing flavor wafts in the nose.

In contrast, at puffing, as the user performs the inhaling operation, air flows into the first flow path 326 through the air intake hole 124 and the fourth flow path 125 of the aerosol generation unit. At this time, a first airflow 362 to flow toward the first inhalation hole 351 is generated in the first flow path 326. The aerosol generated from the aerosol source is urged to flow to the first inhalation hole 351 while accompanied by the first airflow 362. At the same time, since the pressure inside the flavor source storage section 341 becomes a negative pressure by the user inhaling through the filter (third flow path) 343 serving as a second inhalation hole, a second airflow 363 by which external air flows to the flavor source storage section 341 through the second flow path 344 is generated. The flavor generated from the flavor source in the flavor source storage section 341 is thereby urged to flow to the other end through the filter 343, which is the third flow path, while accompanied by the second airflow 363. As a result, since the aerosol and the flavor pass through the first inhalation hole 351 and the second inhalation hole and are inhaled in the user's oral cavity and since the second airflow 363 is inhaled into the user's oral cavity through the filter 343, the flavor source is prevented from flowing into the user's oral cavity.

Therefore, according to the third embodiment, similarly to the first embodiment, the flavor inhaler has the advantage of stimulating the user's olfactory sense by the flavor at non-puffing and stimulating the user's senses of taste and smell by the flavor at puffing.

### (Fourth Embodiment)

A non-combustion flavor inhaler according to a fourth embodiment will be described with reference to FIG. 6. FIG. 6 is a cross-sectional view showing a flavor generation unit of an atomizer unit of the flavor inhaler. The flavor inhaler according to the fourth embodiment is the same as the flavor inhaler shown in FIG. 5 explained in the third embodiment, except for further comprising a second heating element. The same members as those in FIG. 5 are denoted by the same reference numerals in FIG. 6 and their explanations are omitted.

A flavor generation unit 340 comprises a second heating element 370 to heat a flavor source in the flavor source storage section 341. The second heating element 370 is, for example, in a cylindrical shape and disposed so as to be in contact with the inner peripheral surface corresponding to most part of the flavor source storage section 341 of the first partition wall 323 which partitions the flavor source storage section 341. The above-explained power supply 10 is connected to the second heating element 370.

The second heating element 370 is heated to a temperature corresponding to the type of flavor source in the flavor source storage section 341 and is heated to, for example, a temperature lower than the first heating element 80 (room temperature to 250°C). For example, when the power supply 10 is in the on state and the sensor 20 detects an inhaling operation and the detection signal is output to the control unit, and the control unit outputs a control signal to the power supply 10, the second heating element 370 is heated.

The second heating element 370 is preferably configured to be turned on and off by a user's operation. Preferably, the second heating element 370 is configured to be able to operate the applied voltage and to adjust the heating temperature by, for example, operating a dial further provided in the flavor inhaler.

The flavor source has various aspects explained below.
(1) A flavor source similar to that described in the first embodiment can be used.
(2) As a flavor source, a capsule of a heat crushable material containing a flavoring agent can be used. A plurality of capsules having different temperatures for heat crushing can be used together and plural types of flavoring agents contained in the capsules can be used together.
(3) The flavor source may be a mixture of a liquid crushable capsule containing a flavoring agent and an adsorbent releasing a liquid such as water by heating.
(4) The flavor source may contain a fragrance precursor which is thermally decomposed in accordance with the heating temperature of the second heating element 370 to generate a flavor. Examples of the fragrance precursor are, for example, plant origin, plant extract, glycoside, ester, thermally unstable compound having high molecular weight (melanoidin, glycoprotein, and the like).
(5) The flavor source may be an adsorbent having different thermal desorption rates, which is impregnated with a flavoring agent. As the adsorbent, for example, a resin adsorbent such as polystyrene, activated carbon or an inorganic mineral can be used.
(6) The flavor source may be a polymer having a functional group which can be thermally decomposed to become a flavor. Examples of the polymer are, for example, a cellulose, polyvinyl alcohol, an acrylic acid polymer obtained by supporting a perfume component having a carboxylic acid alcohol or an alcohol via an ester bond or ether bond.

Next, the operation of the flavor inhaler according to the fourth embodiment having the above-mentioned configuration will be described with reference to FIG. 6.

The user turns on a power supply 10 of the flavor inhaler with a switch 30 and uses it with holding the mouthpiece end side in the mouth. For example, when the switch is in the on state, a control signal is output from the control circuit 50 to the second heating element 370 to heat the second heating element 370.

At non-puffing, the flavor flows to the outside from the flavor source stored in the flavor source storage section 341 through the second flow path 344. At this time, the amount of generation and the components of the flavor generated from the flavor source can be adjusted by the heating temperature of the second heating element 370. A user holding the flavor inhaler in the mouth can taste the adjusted flavor with the sense of smell since the flowing flavor wafts in the nose.

In contrast, at puffing, the aerosol generated from the aerosol source is urged to flow to the first inhalation hole 351 while accompanied by the first airflow 362, in accordance with the user's inhaling movement. At the same time, the flavor generated from the flavor source in the flavor source storage section 341 is thereby urged to flow to the other end through the filter 343, which is the third flow path, while accompanied by the second airflow 363. As a result, the aerosol and the flavor pass through the first inhalation hole 351 and the second inhalation hole and are inhaled in the user's oral cavity. At this time, the amount of generation and the components of the flavor generated from the flavor source can be adjusted by the heating temperature of the second heating element 370. For this reason, a user holding a flavor inhaler in the mouth can taste the aerosol and the adjusted optimum amount of flavor with the senses of taste and smell.

Therefore, according to the fourth embodiment, similarly to the first embodiment, the flavor inhaler has the advantage of stimulating the user's sense of smell by the flavor at non-puffing and stimulating the user's senses of taste and smell by the flavor at puffing.

The components and amount of generation of the flavor from the flavor source in the flavor source storage section can be adjusted by adjusting the heating temperature of the second heating element 370 and combining the aspects (1) to (6) describing the flavor source.

Since the second heating element 370 is disposed in the first flow path 326, the second heating element 370 can heat the flavor source and heat the aerosol accompanying the first airflow 362. The second heating element 370 can adjust the temperature of the first airflow 362 and reduce the particle diameter of the aerosol accompanying the first airflow 362 by heating the first airflow 362.

The stability of placing the flavor source can be improved. That is, even if the flavor generated from the flavor source is a highly volatile component or a degradable component, the flavor can be held without leaking or being deteriorated until heated by the second heating element 370.

Furthermore, the flavor source storage section 341 of the flavor inhaler according to the fourth embodiment can store the flavor source of components which are destroyed at the temperature of the first heating element 80 for generating the aerosol but are not destroyed at the heating temperature of the second heating element 370. As a result, the user can inhale various flavors.

The second heating element does not need to be formed in a cylindrical shape on the inner peripheral shape of the first partition wall but needs only to heat the flavor source. The second heating element may be disposed in, for example, the flavor source storage section.

### (Fifth Embodiment)

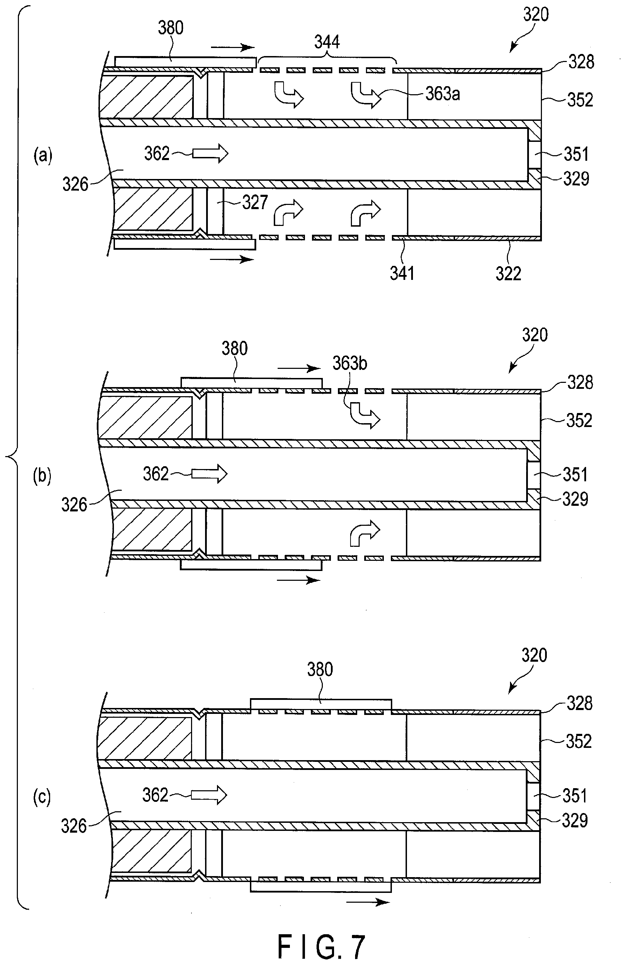

A flavor inhaler according to a fifth embodiment will be described with reference to FIG. 7. FIG. 7 is a cross-sectional view showing a flavor generation unit of an atomizer unit of the flavor inhaler. The flavor inhaler according to the fifth embodiment is the same as the flavor inhaler shown in FIG. 5 explained in the third embodiment, except for further comprising a shutter. The same members as those in FIG. 5 are denoted by the same reference numerals in FIG. 7 and their explanations are omitted.

The flavor generation unit 340 comprises a cylindrical shutter 380 which is provided to be slidable on the second housing 322 and which can open or close the second flow path 344 opened to the second housing 322. The shutter 380 has, for example, a cylindrical shape along the outer circumferential surface of the second housing 322, and has a length enough to cover the entire surface of the second flow path 344.

The shutter 380 can adjust the amount of flavor generated from the flavor source in the flavor source storage section 341 by the sliding operation shown in (a) of FIG. 7 to (c) of FIG. 7 explained below.

As shown in (a) of FIG. 7, when the shutter 380 is retracted, the second flow path 344 is not covered with the shutter 380. That is, the opening area of the second flow path 344 is maximized. For this reason, since a relatively large amount of flavor flows from the second flow path 344, at non-puffing when the user holds the flavor inhaler in the mouth, the flavor greatly stimulates the user's sense of smell. In contrast, since the second flow path 344 is in the most opened state, at puffing when the user holds the flavor inhaler in the mouth and inhales, the rate of the second airflow 363a to the first airflow 362 and second airflow flowing into the oral cavity from the mouthpiece end 328, i.e., the rate of the airflow flowing from the second flow path 344 through the flavor source storage section 341 and the filter (third flow path) 343, becomes maximum.

As shown in (b) of FIG. 7, when the shutter 380 is made to advance to the middle of the second flow path 344, the second flow path 344 is half covered with the shutter 380. For this reason, at non-puffing when the user holds the flavor inhaler in the mouth, the flavor which flows from the second flow path 344 and stimulates the user's sense of smell is halved as compared to the state of (a) of FIG. 7. In addition, at puffing when the user holds the flavor inhaler in the mouth and inhales, the rate of the second airflow 363b to the first airflow 362 and the second airflow 363b flowing from the mouthpiece end 328 decreases as compared with the state of (a) of FIG. 7.

When the shutter 380 is made to further advance as shown in (c) of FIG. 7, the second flow path 344 is completely covered with the shutter 380. For this reason, since the flavor does not flow from the second flow path 344, at non-puffing when the user holds the flavor inhaler in the mouth, the flavor does not stimulate the user's sense of smell. Since only the first airflow 362 accompanying the aerosol flows into the oral cavity from the mouthpiece end, at puffing when the user holds the flavor inhaler in the mouth and inhales, the user can taste only the aerosol. In addition, the flavor source in the flavor source storage section 341 can be prevented from unnecessarily diffusing to the surroundings, by closing with the shutter 380 when not using the flavor inhaler.

Therefore, stimulation of the user's sense of smell caused by the flavor can be adjusted at non-puffing, and stimulation of the user's senses of taste and smell caused by the flavor can be adjusted at puffing, by providing the shutter 380 slidably on the second housing 322 to open and close the second flow path 344.

The flavor generation unit may comprise a second heating element similarly to the flavor generation unit according to the fourth embodiment shown in FIG. 6. By controlling the opening and closing state of the shutter and the heating temperature of the second heating element, the sense of smell can be stimulated at non-puffing by the flavor and the stimulation of the senses of taste and smell can be adjusted at puffing.

### (Sixth Embodiment)

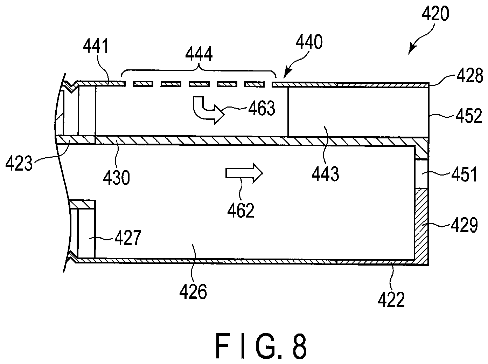

A flavor inhaler according to a sixth embodiment will be described with reference to FIG. 8. FIG. 8 is a cross-sectional view showing a flavor generation unit of an atomizer unit of a flavor inhaler. The flavor inhaler according to the sixth embodiment is the same as the flavor inhaler shown in FIG. 1 and FIG. 2 explained in the first embodiment, except for the flavor generation unit of the atomizer unit.

The flavor generation unit 440 comprises a second housing 422, and the second housing 422 is partitioned by a third partition wall 430 in a flat shape which reaches the mouthpiece end 428 from the second partition 427. The mouthpiece end 428 is defined by sealing an open end of the second housing 422 with, for example, a wall portion 429 integrated with the housing 422. A wall portion 429 located on the upper side of the second housing 422, which is partitioned by the third partition wall 430 in a flat shape, is opened in the substantially semi-columnar hole 452. The inside of the second housing 422 on the lower side of the second housing 422, which partitioned by the third partition wall 430 in the flat shape, a first flow path 426 defined by the inside of the first partition wall 423, the third partition wall 430, and the second housing 422 is formed. In the second housing 422 on the upper side of the second housing 422 partitioned by the third partition wall 430 in the flat shape, a substantially semi-columnar flavor source storage section 441 and a substantially semi-columnar filter 443 are arranged in this order toward the mouthpiece end 428. That is, the flavor source storage section 441 and the semi-columnar filter 443 are arranged so as to be positioned on the user's nose side when the user holds the flavor inhaler in the mouth. The flavor source storage section 441 has a semi-columnar shape in which, for example, an outer wall is defined by the second housing 422, the inner wall is defined by the third partition wall 430, the end on the second connection portion side is defined by the second partition wall 427 and the end on the mouthpiece end side is defined by the filter 433, and flavor source is stored. The filter 443 has one end located on the substantially semi-columnar flavor source storage section 441 and the other end located in the substantially semi-columnar hole 452 of the wall section 429, and constitutes a third flow path extending from the inside of the flavor source storage section 441 to the mouthpiece end 428.

The second flow path 444 including a plurality of holes communicating with the outside is provided in the second housing 422 corresponding to the flavor source storage section 441. A first inhalation hole 451 to allow a user to inhale is opened at a portion opposed to the first flow path 426 of a wall portion 429 defining the mouthpiece end 428 of the second housing 422. The other end of the filter 443 located in the substantially semi-columnar hole of the wall portion 429 functions as a second inhalation hole.

According to the sixth embodiment, since the flavor flows to the outside from the flavor source in the flavor source storage section 441 through the second flow path 444 at non-puffing, the flavor can stimulate only the user's sense of smell.

On the other hand, at puffing, the aerosol generated from the aerosol source is accompanied by the first airflow 462 flowing from the aerosol source through the first flow path 426, and is inhaled into the user's oral cavity through the first inhalation hole 451. At the same time, the flavor generated from the flavor source is accompanied by the second airflow 463 flowing from the second flow path 444 through the flavor source storage section 441 and the filter 443, and is inhaled into the user's oral cavity through the second inhalation hole at the other end of the filter 443.

Therefore, according to the sixth embodiment, similarly to the first embodiment, the flavor inhaler has the advantage of stimulating the user's sense of smell by the flavor at non-puffing and stimulating the user's senses of taste and smell by the flavor at puffing.

In the non-combustion flavor inhaler according to the first embodiment shown in FIG. 1 to FIG. 3, the second heating element to heat the inside of the flavor source storage section may be provided similarly to the flavor inhaler according to the fourth embodiment shown in FIG. 6, or a slidable shutter may be provided similarly to the flavor inhaler according to the fifth embodiment shown in FIG. 7.

In addition, the diameter and the number of the plurality of holes constituting the flow path in the first to sixth embodiments are appropriately selected, based on the properties of the flavor source stored in the flavor source storage section, for example, the strength of flavor, evaporativity, and the like.

While certain embodiments have been described, these embodiments have been presented by way of example only, and are not intended to limit the scope of the inventions. Indeed, the embodiments described herein may be embodied in a variety of other forms; furthermore, various omissions, substitutions and changes in the form of the embodiments described herein may be made without departing from the scope of the invention as defined by the appended claims. The accompanying claims and their equivalents are intended to cover such forms or modifications as would fall within the scope of the invention.

### Reference Signs List

10 ... power supply, 20 ... sensor, 30 ... switch, 40 ... light emitting element, 50 ... control circuit, 60 ... holding body, 70 ... absorber, 80 ... aerosol generation mechanism (first heating element), 100 ... non-combustion flavor inhaler, 110 ... power supply unit, 111 ... first connection portion, 112 ... first housing, 120, 220, 320, 420 ... atomizer unit, 121 ... second connection unit, 121a ... recess, 122, 222, 322, 422 ... second housing, 123, 223, 323, 423 ... first partition wall, 124 ... air intake hole, 125 ... fourth flow path, 126, 226, 326, 426 ... first flow path, 127, 227, 327, 427 ... second partition wall, 128, 228, 328, 428 ... mouthpiece end, 129, 229, 329, 429 ... wall portion, 130 ... aerosol generation unit, 131 ... aerosol source storage section, 140, 240, 340, 440 ... flavor generation unit, 141, 241, 341, 441 ... flavor source storage section, 343, 443 ... filter, 144, 244, 344, 444 ... second flow path, 145 ... third flow path, 151, 251, 351, 451 ... first inhalation hole, 252 ... second inhalation hole, 161 ... flavor, 162, 262, 362, 462 ... first airflow, 163, 263, 363, 463 ... second airflow, 370 ... second heating element, 380 ... shutter, 430 ... third partition wall.

## Claims

1. A non-combustion flavor inhaler (100) comprising an aerosol generation unit (130), a flavor generation unit (140), and a mouthpiece end (128),
the aerosol generation unit (130) comprising an aerosol source storage section (131) for storing an aerosol source and an aerosol generation mechanism (80) for generating an aerosol from the aerosol source,
the flavor generation unit (140) comprising a flavor source storage section (141) for storing a flavor source,
the aerosol generation unit (130) being provided with a first flow path (126) extending from within the aerosol generation unit (130) to the mouthpiece end (128),
the flavor source storage section (141) being provided with a second flow path (144) consisting of at least one hole communicating with outside, and a third flow path (145) extending from within the flavor source storage section (141) to the first flow path (126),
**characterized in that**
the third flow path (145) extending to the first flow path (126) is at least one hole which communicates the first flow path (126) with the inside of the flavor source storage section (141).

2. The non-combustion flavor inhaler (100) according to Claim 1, wherein the aerosol generation mechanism (80) is a first heating element (80) which heats the aerosol source.

3. The non-combustion flavor inhaler (100) according to Claim 2, wherein a temperature of the first heating element (80) is adjustable.

4. The non-combustion flavor inhaler (100) according to Claim 2 or 3, wherein the first heating element (80) is capable of being turned on and off.

5. The non-combustion flavor inhaler (100) according to any one of Claims 2 to 4, wherein the flavor generation unit (130) further comprises a second heating element (370) which heats the flavor source in the flavor source storage section (141).

6. The non-combustion flavor inhaler (100) according to Claim 5, wherein the first heating element (80) is heated at a higher temperature than the second heating element (370).

7. The non-combustion flavor inhaler (100) according to Claim 5 or 6, wherein a temperature of the second heating element (370) is adjustable.

8. The non-combustion flavor inhaler (100) according to any one of Claims 5 to 7, wherein the second heating element (370) is capable of being turned on and off.

9. The non-combustion flavor inhaler (100) according to any one of Claims 1 to 8, wherein a flavor-permeable member is provided in at least one hole constituting the second flow path (144).

10. The non-combustion flavor inhaler (100) according to any one of Claims 1 to 9, further comprising a shutter (380) which opens or closes at least one hole constituting the second flow path (144).

## Patentansprüche

1. Verbrennungsfreier Aromainhalator (100) umfassend eine Aerosolerzeugungseinheit (130), eine Aromaerzeugungseinheit (140), und ein Mundstückende (128),
wobei die Aerosolerzeugungseinheit (130) einen Aerosolquellenaufbewahrungsabschnitt (131) zum Aufbewahren einer Aerosolquelle und einen Aerosolerzeugungsmechanismus (80) zum Erzeugen eines Aerosols aus der Aerosolquelle umfasst,
wobei die Aromaerzeugungseinheit (140) einen Aromenquellenaufbewahrungsabschnitt (141) zum Aufbewahren einer Aromenquelle umfasst,
wobei die Aerosolerzeugungseinheit (130) mit einem ersten Strömungsweg (126) versehen ist, der sich vom Inneren der Aerosolerzeugungseinheit (130) zum Mundstückende (128) erstreckt
wobei der Aromaquellenaufbewahrungsabschnitt (141) mit einem zweiten Strömungsweg (144) versehen ist, der aus mindestens einem Loch besteht, das nach außen führt, und einem dritten Strömungsweg (145), der sich vom Inneren des Aromaquellenaufbewahrungsabschnitts (141) zu dem ersten Strömungsweg (126) erstreckt,
**dadurch gekennzeichnet, dass**
der dritte Strömungsweg (145), der sich zu dem ersten Strömungsweg (126) erstreckt, mindestens ein Loch ist, das den ersten Strömungsweg (126) mit dem Inneren des Aromaquellenaufbewahrungsabschnitts (141) verbindet.

2. Verbrennungsfreier Aromainhalator (100) nach Anspruch 1, wobei der Aerosolerzeugungsmechanismus (80) ein erstes Heizelement (80) ist, das die Aerosolquelle erwärmt.

3. Verbrennungsfreier Aromainhalator (100) nach Anspruch 2, wobei eine Temperatur des ersten Heizelements (80) einstellbar ist.

4. Verbrennungsfreier Aromainhalator (100) nach Anspruch 2 oder 3, wobei das erste Heizelement (80) ein- und ausgeschaltet werden kann.

5. Verbrennungsfreier Aromainhalator (100) nach einem der Ansprüche 2 bis 4, wobei die Aromaerzeugungseinheit (130) ferner ein zweites Heizelement (370) umfasst, das die Aromaquelle in dem Aromaquellenaufbewahrungsabschnitt (141) erhitzt.

6. Verbrennungsfreier Aromainhalator (100) nach Anspruch 5, wobei das erste Heizelement (80) auf eine höhere Temperatur als das zweite Heizelement (370) erhitzt wird.

7. Verbrennungsfreier Aromainhalator (100) nach Anspruch 5 oder 6, wobei eine Temperatur des zweiten Heizelements (370) einstellbar ist.

8. Verbrennungsfreier Aromainhalator (100) nach einem der Ansprüche 5 bis 7, wobei das zweite Heizelement (370) ein- und ausgeschaltet werden kann.

9. Verbrennungsfreier Aromainhalator (100) nach einem der Ansprüche 1 bis 8, wobei ein aromadurchlässiges Element in mindestens einem Loch vorgesehen ist, das den zweiten Strömungsweg (144) bildet.

10. Verbrennungsfreier Aromainhalator (100) nach einem der Ansprüche 1 bis 9, ferner umfassend einen Verschluss (380), der mindestens ein Loch, das den zweiten Strömungsweg (144) bildet, öffnet oder verschließt.

## Revendications

1. Inhaleur d'arôme sans combustion (100) comprenant une unité de production d'aérosol (130), une unité de production d'arôme (140), et une extrémité d'embout (128),
l'unité de production d'aérosol (130) comprenant une section de stockage de source d'aérosol (131) pour stocker une source d'aérosol et un mécanisme de production d'aérosol (80) pour produire un aérosol à partir de la source d'aérosol,
l'unité de production d'arôme (140) comprenant une section de stockage de source d'arôme (141) pour stocker une source d'arôme,
l'unité de production d'aérosol (130) étant munie d'une première trajectoire d'écoulement (126) s'étendant à partir de l'unité de production d'aérosol (130) jusqu'à l'extrémité d'embout (128),
la section de stockage de source d'arôme (141) étant munie d'une seconde trajectoire d'écoulement (144) consistant en au moins un orifice communiquant avec l'extérieur, et d'une troisième trajectoire d'écoulement (145) s'étendant à partir de l'intérieur de la section de stockage de source d'arôme (141) jusqu'à la première trajectoire d'écoulement (126),
**caractérisé en ce que**
la troisième trajectoire d'écoulement (145) s'étendant jusqu'à la première trajectoire d'écoulement (126) est au moins un orifice qui fait communiquer la première trajectoire d'écoulement (126) avec l'intérieur de la section de stockage de source d'arôme (141).

2. Inhaleur d'arôme sans combustion (100) selon la revendication 1, dans lequel le mécanisme de production d'aérosol (80) est un premier élément de chauffage (80) qui chauffe la source d'aérosol.

3. Inhaleur d'arôme sans combustion (100) selon la revendication 2, dans lequel une température du premier élément chauffant (80) est ajustable.

4. Inhaleur d'arôme sans combustion (100) selon la revendication 2 ou 3, dans lequel le premier élément chauffant (80) peut être allumé ou éteint.

5. Inhaleur d'arôme sans combustion (100) selon l'une quelconque des revendications 2 à 4, dans lequel l'unité de production d'arôme (130) comprend de plus un second élément chauffant (370) qui chauffe la source d'arôme dans la section de stockage de source d'arôme (141).

6. Inhaleur d'arôme sans combustion (100) selon la revendication 5, dans lequel le premier élément chauffant (80) est chauffé à une température plus élevée que le second élément chauffant (370).

7. Inhaleur d'arôme sans combustion (100) selon la revendication 5 ou 6, dans lequel une température du second élément chauffant (370) est ajustable.

8. Inhaleur d'arôme sans combustion (100) selon l'une quelconque des revendications 5 à 7, dans lequel le second élément chauffant (370) peut être allumé ou éteint.

9. Inhaleur d'arôme sans combustion (100) selon l'une quelconque des revendications 1 à 8, dans lequel un élément perméable à l'arôme est fourni dans au moins un orifice constituant la seconde trajectoire d'écoulement (144).

10. Inhaleur d'arôme sans combustion (100) selon l'une quelconque des revendications 1 à 9, comprenant de plus un volet (380) qui ouvre ou ferme au moins un orifice constituant la seconde trajectoire d'écoulement (144).
